(19) **Europäisches Patentamt / European Patent Office / Office européen des brevets**

(11) **EP 3 892 997 A1**

(12) # EUROPEAN PATENT APPLICATION

(43) Date of publication:
**13.10.2021 Bulletin 2021/41**

(51) Int Cl.:
**G01N 33/53** (2006.01)        **G01N 33/48** (2006.01)
**G01N 33/68** (2006.01)

(21) Application number: **21166730.8**

(22) Date of filing: **10.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.01.2013   US 201361751107 P**
**14.03.2013   US 201361784151 P**

(62) Document number(s) of the earlier application(s) in accordance with Art. 76 EPC:
**14737727.9 / 2 943 904**

(71) Applicants:
• **Emory University**
  **Atlanta, Georgia 30322 (US)**
• **Georgia Tech Research Corporation**
  **Atlanta, Georgia 30332-0415 (US)**

(72) Inventors:
• **DOETSCH, Paul W.**
  **Atlanta, GA 30345 (US)**

• **HUNT, William D.**
  **Atlanta, GA 30332-0250 (US)**
• **SHIN, Dong Moon**
  **Atlanta, GA 30345 (US)**
• **CHEN, Georgia Zhuo**
  **Lawrenceville, GA 30044 (US)**
• **MORENO, Carlos Sanchez**
  **Atlanta, GA 30327 (US)**

(74) Representative: **Lau, Sarah Jane et al**
**Kilburn & Strode LLP**
**Lacon London**
**84 Theobalds Road**
**London WC1X 8NL (GB)**

Remarks:
This application was filed on 01.04.2021 as a divisional application to the application mentioned under INID code 62.

(54) **SYSTEMS, METHODS AND COMPUTER READABLE STORAGE MEDIA FOR ANALYZING A SAMPLE**

(57)    Methods, systems, and computer readable storage media relate to processing and analyzing a sample to determine presence or concentration of at least one sensor target in a sample. A method of quantifying a sample using a sensor may include obtaining sensor data, the sensor data including in-situ calibration data from a housekeeping protein; analyzing the sensor data; and outputting results of the analyzing. The method may be used with a system that includes a sensing device and a module. The system may also include an analysis device that can be in communication with the sensing device.

FIGURE 1

EP 3 892 997 A1

**Description**

CROSS REFERENCE TO RELATED APPLICATIONS

This application claims priority to Provisional Application Serial Number 61/751,107 filed January 10, 2013 and Provisional Application Serial Number 61/784,151 filed March 14, 2013, which are hereby incorporated by reference in their entirety.

**BACKGROUND**

Immunosensing is a field of study focused on the development of devices that detect target antigens based on the high affinity reactions to antibodies within a sensing platform. The antibody-antigen interaction was first exploited by immunoassay processes such as enzyme linked immunosorbent assay (ELISA) and later the home pregnancy test.

Immunosensors can be generally categorized into 4 groups based on their detection mechanism - electrochemical, optical, magnetic, or piezoelectric. See, e.g., Holford, T. R., Davis, F., & Higson, S. P. (2012). Recent trends in antibody based sensors. Biosensors and Bioelectronics, 34(1), 12-24. Piezoelectric sensors have been used to study the properties of a broad range of biochemical reactions. An emerging application of such sensors is the detection of biomarkers.

Accuracy and efficiency are two critical components for immunosensor systems. This is typically addressed with the selection of surface chemistry to minimize factors such as non-specific binding. However, there are still many different factors that can alter results, such as, sample preparation, sensor environment, and human error.

**SUMMARY**

Thus, there is a need for immunosensor systems, devices, and methods that accurately and efficiently detect target antigens by addressing these factors.

The application relates to methods, systems, and computer readable storage media for processing and analyzing samples to determine presence or concentration of at least one biomarker in a sample.

In some embodiments, the disclosure may relate to a method of quantifying a sample using a sensor. The method may include obtaining sensor data, the sensor data including *in-situ* calibration data from a housekeeping protein; analyzing the sensor data; and outputting results of the analyzing. The method may be performed automatically by a device having a processor and a memory.

In some embodiments, the analyzing the sensor data may include determining quantitative values from the sensor data; and comparing the quantitative values to a classification model. The determining the quantitative values may include determining frequency values.

In some embodiments, the sample may be a blood sample from a subject and the housekeeping protein may be β-Actin.

In some embodiments, the disclosure may relate to a module configured to be used with a sensing device that is configured to analyze a sample. The module may include a chip including at least one sensor target to be tested; at least one fluid connector configured to be in fluid communication with a sensing device; and a connector configured to connect the module to the sensing device.

In some embodiments, the connector may include a USB connector. In some embodiments, the module may include identification information, the identification information including at least one of type of analysis to be performed, operation parameters, and classification model related to analysis. The identification information may include a bar code.

In some embodiments, the disclosure may relate to a sensing device configured to extract sensor data from a sensor module. The sensing device may include at least one port configured to receive a module; a module interface configured to receive and transmit data between the module and the device; and a controller configured to control an operation of the sensing device.

In some embodiments, the disclosure may relate to a system configured to analyze a sample. The system may include a sensing device configured to extract data from at least one module; and at least one module. The sensing device may include at least one port configured to receive a module; a module interface configured to receive and transmit data between the module and the device; and a controller configured to control an operation of the sensing device. The module may include a chip including at least one sensor target to be tested; at least one fluid connector configured to be in fluid communication with the module; and a connector configured to connect the module to the sensing device.

In some embodiments, the system may further include an analysis device configured to perform analysis of the data. In some embodiments, the analysis may include quantification of frequency values.

In some embodiments, the module may include identification information, the identification information including at least one of type of analysis to be performed, operation parameters, and classification model related to the analysis.

In some embodiments, the sensing device may be configured to operate according to the operation parameters. The sensing device may include a characterization board configured to extract sensor data in substantially real time. The sensor data may include frequency values.

In some embodiments, the connector of the module may include a USB connector. The port may be configured to receive the connector and the fluid connector of the module.

Additional advantages of the disclosure will be series forth in part in the description which follows, and in part will be obvious from the description, or may be learned by practice of the disclosure. The advantages of the disclosure will be realized and attained by means of the elements and combinations particularly pointed out in the appended claims. It is to be understood that both the foregoing general description and the following detailed description are exemplary and explanatory only and are not restrictive of the disclosure, as claimed.

## BRIEF DESCRIPTION OF THE DRAWINGS

The disclosure can be better understood with the reference to the following drawings and description. The components in the figures are not necessarily to scale, emphasis being placed upon illustrating the principles of the disclosure.

Figure 1 shows a method of analyzing a sample according to embodiments.

Figure 2 shows a method of classifying the sensor data according to embodiments.

Figure 3 shows an exemplary method of generating classification models according to embodiments.

Figure 4 shows an exemplary method of operation of a system according to embodiments.

Figure 5 shows another exemplary method of operation of a system according to embodiments.

Figure 6 shows an example of a system according to embodiments.

Figure 7 shows a perspective view of a sensor module according to embodiments.

Figure 8 shows another perspective view of the sensor module shown in Figure 7.

Figure 9 shows an enlarged view of the sensor module shown in Figures 7 and 8.

Figures 10A and B show a sensor device without a sensor module and a sensor device with a sensor module according to embodiments.

Figures 11A and 11B show frequency shift plots for all harmonics of one lysate sample trial according to one example. (A) Maximum to minimum, (b) total frequency shift, (c) binding affinity, (d) equilibrium, (e) dissociation regions slope values.

Figure 12 shows an example mean and standard deviation values for binding affinity region slope values, (a) shows a desired data set separation and (b) shows an undesired case where there is no statistical separation between the sample types.

Figure 13 shows a 2D model based on the binding affinity region slope values.

Figure 14 shows a 3D model plot.

Figure 15 shows a Bayesian classifier based on Gaussian Mixture Models (GMM).

## DETAILED DESCRIPTION OF THE EMBODIMENTS

The following description, numerous specific details are series forth such as examples of specific components, devices, methods, etc., in order to provide an understanding of embodiments of the disclosure. It will be apparent, however, to one skilled in the art that these specific details need not be employed to practice embodiments of the disclosure. In other instances, well-known materials or methods have not been described in detail in order to avoid unnecessarily obscuring embodiments of the disclosure. While the disclosure is susceptible to various modifications and alternative forms, specific embodiments thereof are shown by way of example in the drawings and will herein be described in detail. It should be understood, however, that there is no intent to limit the disclosure to the particular forms disclosed, but on the contrary, the disclosure is to cover all modifications, equivalents, and alternatives falling within the spirit and scope of the disclosure.

This disclosure generally relates to methods, systems, and computer readable storage media for processing samples to determine presence or concentration of at least one biomarker in a sample. The concentrations of the biomarker can be useful, for example, diagnosis as well as evaluating treatment effectiveness. A single sample can be used to process, determine and quantify at least one biomarker without a specific preparation of the sample.

In some embodiments, the disclosure can utilize a classification system with techniques for normalizing the data. In some embodiments, the disclosure can calibrate in-situ, for example, by using a housekeeping protein. The sample can be processed and analyzed under computer control and thereby can significantly eliminate potentially user error.

The system can also be capable of detecting and measuring a panel of biomarkers simultaneously from a single sample. It can thereby quickly provide the clinician and patient with critical clinical information regarding diagnosis, resulting in a personalized treatment decisions that are correlated with good clinical outcomes. Thus, the methods, systems, and computer readable storage media according to the disclosure addresses the factors that can alter results, such as sample preparation, sensor environment, and human error.

## Terms

Unless defined otherwise, all technical and scientific terms used herein have the same meaning as is commonly understood by one of ordinary skill in the art to which this disclosure belongs. In the event that there is a plurality of definitions for a term herein, those in this section prevail unless stated otherwise.

It is also to be understood that the terminology used herein is for the purpose of describing particular embodiments only, and is not intended to be limiting, since the scope of the present disclosure will be limited only by the appended claims.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure.

Embodiments of the present disclosure will employ, unless otherwise indicated, techniques of synthetic organic chemistry, biochemistry, biology, molecular biology, pharmacology, and the like, which are within the skill of the art. Such techniques are explained fully in the literature.

It must be noted that, as used in the specification and the appended claims, the singular forms "a," "an," and "the" include plural referents unless the context clearly dictates otherwise. In this specification and in the claims that follow, reference will be made to a number of terms that shall be defined to have the following meanings unless a contrary intention is apparent.

As used herein, "subject" refers to any animal, preferably a human patient, livestock, or domestic pet.

The term "diagnosed," as used herein, refers to the identification of the presence or nature of a pathological condition, for example, a disease, (e.g., caused by the presence of pathogenic bacteria) by its signs and symptoms (e.g., resistance to conventional therapies), or genetic analysis, pathological analysis, histological analysis, and the like.

"Detect" can refer to the identification of the presence, absence or the amount of the target molecule in the sample.

"Sample" refers to any non-organic solution that can contain the target molecule. A sample may be blood, plasma, serum, urine, food containing solution, water containing pollutants etc. The target molecule contained in the sample may be polypeptides, peptides, proteins, antibodies, cells, chemicals etc.

"Biosensor" or "sensor" refers to a small, portable, analytical device based on the combination of capturing molecules with an appropriate transducer. The device can detect chemical or biological materials selectively and with high sensitivity. A "biosensor" can include but is not limited to an electrochemical, optical, magnetic, or piezoelectric biosensors or immunosensors.

The phrase "development or tendency of development" refers to the status of the patient for the likelihood of developing certain disease based on the result obtained from the biosensor.

"Sensor target" generally refers to a protein or other molecules which is acted upon by constituents present in a sample taken from patients having or having the tendency of developing cancer or other kind of diseases or food sample containing pathogens, or water sample containing pollutants as compared to sample taken from control subjects (e.g., healthy subject, clean food, or unpolluted water solution).

## Methods of Detecting Biomarkers

The methods of the disclosure are not limited to the steps described herein. The steps may be individually modified or omitted, as well as additional steps may be added.

Unless stated otherwise as apparent from the following discussion, it will be appreciated that terms such as "averaging," "calibrating," "filtering," "combining," "analyzing," "comparing," "generating," "determining," "obtaining," "processing," "computing," "selecting," "estimating," "detecting," "outputting," "applying," "classifying," "extracting," "receiving," or "acquiring," or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods may be compiled for execution on a variety of hardware platforms and for interface to a variety of operating systems. In addition, embodiments are not described with reference to any particular programming language. It will be appreciated that a variety of programming languages may be used to implement embodiments of the disclosure.

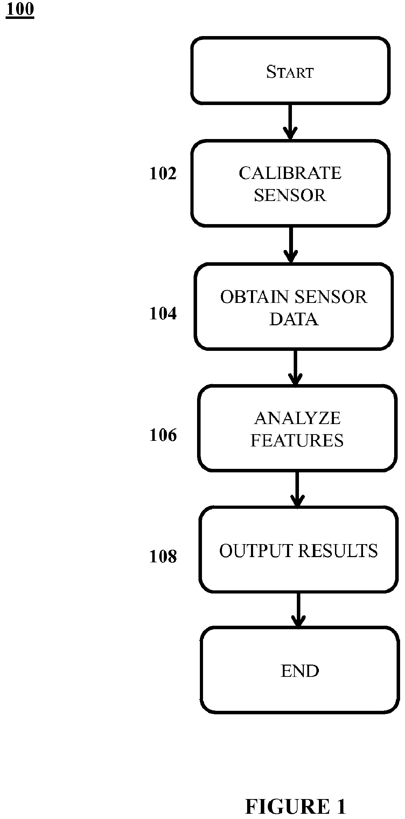

Figure 1 shows a method 100 of analyzing a sample according to embodiments. After a sample is loaded, the method 100 may include a step 102 of calibrating a sensor before analyzing the features (sensor data) of the sample. The calibrating step 102 may include performing in-situ calibration of the sensor system in which the sample is loaded. The calibrating step 102 may include using a housekeeping protein. The housekeeping protein may be specific to the sample to be tested and/or sensor target. The housekeeping protein may be any protein that has an expression pattern that has relatively constant levels between both positive and negative cell types. For example, for a blood sample, the housekeeping protein may include β-Actin.

By using a calibrating step, the concentration of biomarkers present can be accurately quantified. In order to ensure binding has occurred, a buffer solution can be caused to be flown across the sensor after the lysate to wash away any molecules not bound to a sensor target. The lower the frequency value the greater the concentration of that specific protein in the lysate sample.

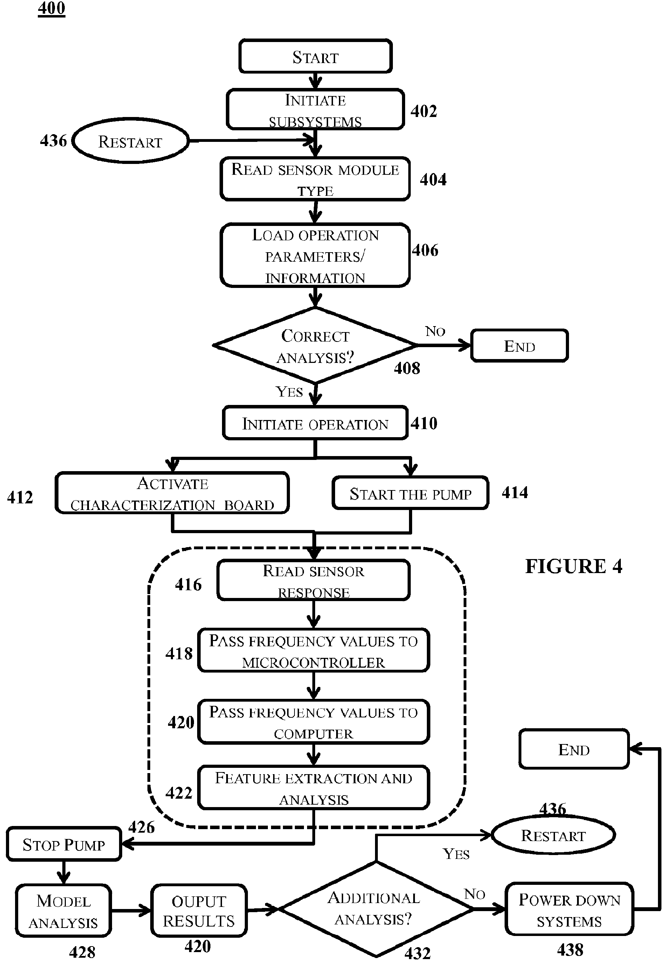

In some embodiments, the calibrating step 102 may be performed when the sample is caused to be drawn through the sensor system for analysis. After the sensor is calibrated (e.g., the sample is drawn through the sensor system), the sensor data may be obtained (step 104). The sensor data may include *in-situ* calibration data. After the sensor data is obtained (for example, according to method shown in Figure 4), the sensor data may be analyzed for specific features (step 106). The features may be specific to the biomarker(s) or disease state(s) to be analyzed.

Based on the selected sensor module and associated operation, the acquired data can be analyzed. In some embodiments, the acquired data may be compared to the respective classification model. In some embodiments, the data may be analyzed to determine a certain classification (i.e. patient has likelihood for a disease state), as well as other types of information (i.e. biomarker structure, presence of carcinogen, etc.).

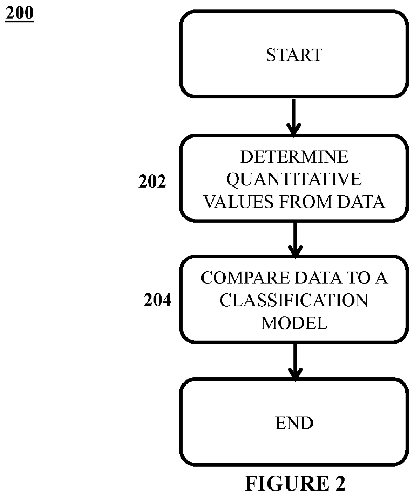

Figure 2 shows an example of an analysis method 200. As shown in Figure 2, the analysis may include a step 202 of determining quantitative values from the sensor data for specific features.

The quantitative values may include information, such concentration information (e.g., ng/ml) for a particular marker. The determining step 202 may include determining or tracking the change in the sensor, for example, for piezoelectric sensors, this would include a shift in the device's fundamental and harmonic frequencies.

In some embodiments, the quantitative values may include determining the frequency shift of the total bonded antigen for a predetermined time period. The time period may include the period from the initial frequency to after sensor target immobilization to the final buffer wash off.

After the values are determined, the values for the features may be compared to a classification model to classify and/or analyze the biomarker(s) (step 204). In some embodiments, the classification model may include disease state (e.g., likelihood). The classification model may be prestored and be specific to the specific biomarker(s) or disease state(s) to be analyzed.

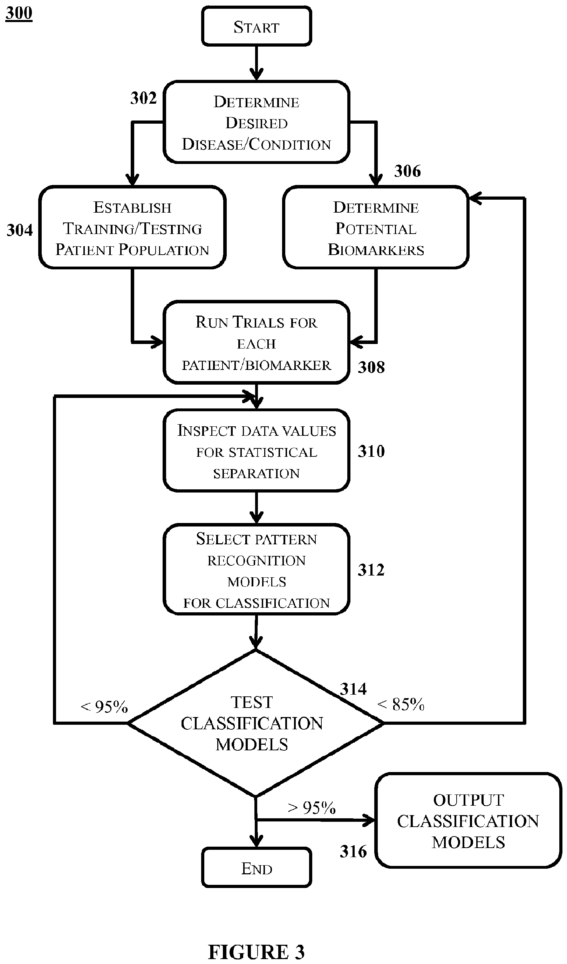

Figure 3 shows a method 300 of determining a classification model according to some embodiments. In other embodiments, the stored classification model may be determined according to other techniques.

In some embodiments, the method 300 may include a step 302 of determining the desired disease/condition for the classification model. In some embodiments, the method may include a step 304 of establishing a training/testing population. Patient population may be selected to represent all aspects of the disease similar to those selected for clinical research journals, for example, taking into account diversity, other diseases, etc. The population may be separated into a training set for developing the model and a testing set for checking each model's accuracy.

The method 300 may include a step 306 of determining potential biomarkers. For example, selection of potential biomarkers can be based on previous research conducted for the disease and the final list will be determined by expert opinion.

The method may include a step 308 of running trials for each patient/biomarker.

Next the data values may be analyzed for statistical separation (step 310). Feature extraction can be similar for all trials. For example, each trial can have a max-min and regular shift and slope values for each of the three regions explained in the publication. After obtaining each feature values from the trials, the selection of which values can be used to develop classification models can be manually or automatically determined.

In step 312, pattern recognition models for classification may be selected. After determining the desired feature values, all of the training set values can be separated with different types of pattern recognition algorithms (developed by others research laboratories), such as linear or Bayesian classifiers. This can result in either single or multiple classification models for classifying each patient.

The models may then be tested (step 314). Each model may then evaluated with the testing set of patients selected in the same way as the training set but not utilized for model development. Depending on a predetermined classification accuracy, the model can either complete or be ready for utilization with the sensors and systems (e.g., greater than about 95%), need reselection of feature values (e.g., between about 85%-95%), and/or reassessment of patient population and biomarkers (less than about 85%). The predetermined classification accuracy is not limited to those thresholds shown in Figure 3 and may be different.

If the model is above the predetermined classification accuracy, the classification model may be outputted (step 316). For example, the classification model may be stored for later use with the system. In some embodiments, the classification model may be stored with the additional information, for example, operation parameters associated with the disease/condition to be analyzed.

After the features are analyzed (e.g., classified according to the classification model), the results of the classification may then be outputted (step 108). In some embodiments, the outputting may include displaying, printing, storing, and/or transmitting the results. In some embodiments, the results may be transmitted to another system, server and/or storage device for the printing, displaying and/or storing the results.

**Module** & **Sensor System**

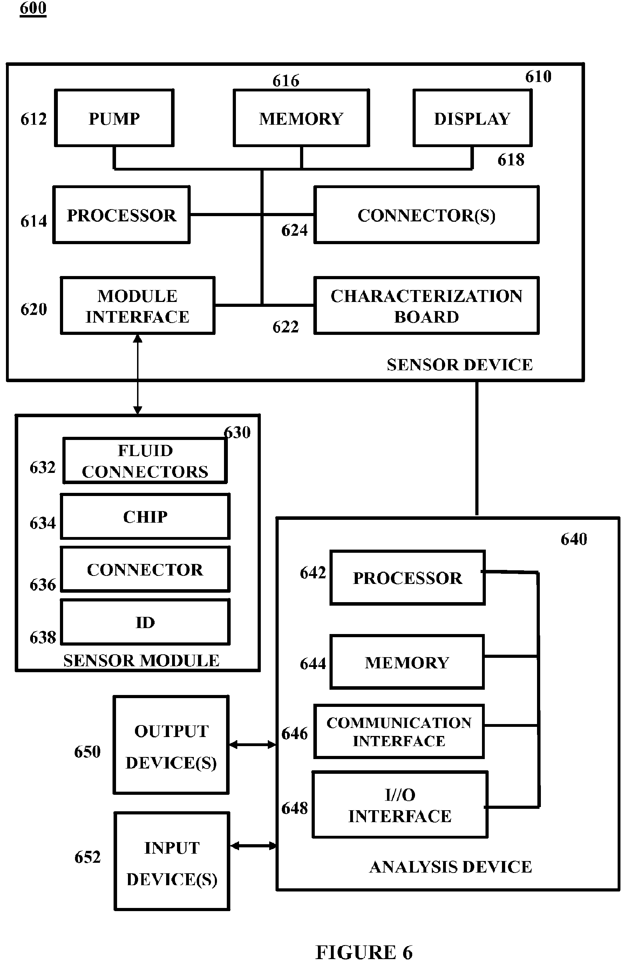

Figure 6 shows an example of a system 600 that may be used to obtain and analyze sensor data according to embodiments. The system 600 may include any number of modules that communicate with other through electrical or data connections (not shown). In some embodiments, the modules may be connected via a wired network, wireless network, or combination thereof. In some embodiments, the networks may be encrypted. In some embodiments, the wired network may be, but is not limited to, a local area network, such as Ethernet, or wide area network. In some embodiments, the wireless network may be, but is not limited to, any one of a wireless wide area network, a wireless local area network, a Bluetooth network, a radio frequency network, or another similarly functioning wireless network.

Although the modules of the system are shown as being directly connected, the modules may be indirectly connected to one or more of the other modules of the system. In some embodiments, a module may be only directly connected to one or more of the other modules of the system.

It is also to be understood that the system may omit any of the modules illustrated and/or may include additional modules not shown. It is also be understood that more than one module may be part of the system although one of each module is illustrated in the system. It is further to be understood that each of the plurality of modules may be different or may be the same. It is also to be understood that the modules may omit any of the components illustrated and/or may include additional component(s) not shown.

In some embodiments, the modules provided within the system may be time synchronized. In further embodiments, the system may be time synchronized with other systems, such as those systems that may be on the medical facility network.

As shown in Figure 6, the system 600 may include a sensor device 610 configured to obtain and read sensor data from a sensor module 630 (also referred to as "module" and "cartridge"). The system 600 may include at least one sensor module 630. In some embodiments, the system 600 may include an analysis device 640 configured to perform analysis of the sensor data.

In some embodiments, the device 610 may be a single-use, disposable device. In other embodiments, the device 610 may be configured to be reusable and sterilized, for example, with a plurality of sensor modules 630. In some embodiments, the device 610 may be configured to be used with other sensor modules.

In some embodiments, a sensor device 610 may include at least one pump 612. The pump 612 may be configured to provide a uniform flow system. The pump 612 may be configured to pull a sample through the device 610. The pump 612 may be a peristaltic pump.

In some embodiments, the device 610 may include one or more of any processing units (also referred to as "processor" or "controller") 614. The processor 614 may be configured to control the system and establish communication to the analysis device 640. The processor 614 or 610 may be a CPU, such as a processor or a microprocessor or microcontroller.

In some embodiments, the device 610 may include a memory 616. The processor 614 may be coupled directly or indirectly to one or more memory (e.g., computer-readable storage medium) 616. The memory 616 may include one or more memory elements, such random access memory (RAM), read only memory (ROM), disk drive, tape drive, etc., or a combinations thereof. The memory 616 may be encoded or embed with computer-readable instructions, which, when executed by one or more processors 614 cause the device 610 and/or system 600 to carry out various functions.

In some embodiments, the device 610 may include a display 618 configured to provide information and status updates of the analysis. The display 618 may be an LED panel. The display 618 may be a part and/or separate from the device 610. In some embodiments, the display 618 may be omitted.

In some embodiments, the device 610 may include a module interface 620. The module interface 620 may be configured to conduct receiving and transmitting of data between the sensor module 630 and other modules of the device 610.

In some embodiments, the device 610 may include a characterization board 622. The characterization board 622 may be configured to extract sensor data (e.g., frequency values) in substantially real time (e.g., about 10+ data points per second) and transmit the data to the processor 614.

The device 610 may include at least one connector 624 configured to connect with one or more connectors of the module 630, another device, and/or a combination thereof. In some embodiments, the at least one connector 624 may include a connector configured to transmit at least data, at least one fluid connector, or any combination thereof. In some embodiments, the connector 624 may include an Universal Serial Bus (USB) compatible connector, for example, a USB port. In some embodiments, the module interface 620 may be configured to interface with the USB port.

In some embodiments, the connector 624 may include at least one fluid connector configured to receive the at least one fluid connector of the sensor module 632. In some embodiments, the connector 624 may include two fluid connectors, such as fluid ports, configured to respectively receive the inlet and outlet connectors of the module 630. In some embodiments, the device 610 may include another connector configured to communicate with a separate analysis device, for example, a computer, and/or display.

In some embodiments, the sensor module 630 may be specific to the biomarker/disease state to be analyzed and/or classified. The system 600 may include a plurality of sensor modules 630, each being for a different biomarker/disease state. The sensor module 630 may be a single-use, disposable device. In other embodiments, the sensor module 630 may be configured to be reusable and sterilized.

In some embodiments, the sensor module 630 may be adapted for use with other sensing devices.

In some embodiments, the sensor module 630 may include at least one sensor target. In some embodiments, the at least one sensor target may be based on the sample to be tested, disease/condition to be tested, or a combination thereof. The sensor target may include but is not limited to one or more antibodies. In some embodiments, the sensor module 630 may include a plurality of antibodies. In some embodiments, the plurality of antibodies may include one or more antibodies specific for at least one housekeeping protein (also referred to as "one or more housekeeping antibodies").

In some embodiments, the sensor module 630 may include at least one sensor chip 634. In some embodiments, the sensor 630 may include more than one chip 634. The chip 634 may include but is not limited to a zinc oxide chip. The chip 634 may include the at least one sensor target. In some embodiments, the chip 634 may include one or more antibodies specific for a housekeeping protein, for example, to allow for in-situ calibration of the module 630. For example, for a blood sample, the housekeeping protein may include β-Actin.

In some embodiments, the sensor chip 634 may include one or more sensors. In some embodiments, the chip 634 may include at least one piezoelectric sensor. In other embodiments, the chip 634 may include other sensors, including but not limited to electrochemical, optical, and/or magnetic biosensor.

In some embodiments, the one or more sensors may be each immobilized with the one or more of the same antibodies, one or more of different antibodies, or any combination thereof. For example, the one or more sensors may be each immobilized with at least one sensor target, one or more housekeeping antibodies, and/or a combination thereof.

In some embodiments, the module 630 may include one or more circuit board. The circuit board any type of circuit board, including but not limited to a printed circuit board. In some embodiments, the sensor chip 634 may be disposed above the electronic circuit.

In some embodiments, the module 630 may include one or more fluid connectors 632. The fluid connectors 632 may include an inlet and an outlet.

The module 630 may include a connector 636 configured to connect to the module 630 to the device 610. In some embodiments, the connector 636 may be a Universal Serial Bus (USB) compatible connector. The USB connector may be a non-digital, digital, mixed signal use, or a combination thereof.

In some embodiments, the module 630 may further include identification information 638. The identification information 638 may include biomarker to be tested. The identification information 638 may be a barcode. In some embodiments, the identification information may be stored along with operation information, for example, in the memory 644 and/or the memory 616. The operation information may include but is not limited to operation parameters for the sensor device, type of analysis to be performed, classification model(s) related to analysis and/or housekeeping protein, or any combination thereof. In some embodiments, the operation information may be specific to the sensor target.

In some embodiments, the analysis device 640 may be a separate device. In other embodiments, the analysis device 640 may be a part (e.g., stored on the memory) of other modules, for example, the sensor device 610, and controlled by its respective CPUs.

The analysis device 640 may be a computing system, such as a workstation, computer, or the like. The analysis device 640 may include one or more processors 642. The processor 642 may be one or more of any central processing units, including but not limited to a processor or a microprocessor. The processor 642 may be coupled directly or indirectly to one or more memory elements (e.g., computer-readable storage medium) 644. The memory elements may include but is not limited to random access memory (RAM), read only memory (ROM), disk drive, tape drive, etc., or a combinations thereof. The memory 644 may be encoded or embedded with computer-readable instructions, which, when executed by one or more processors 642 cause the analysis device 640 and/or device 610 to carry out various functions. In some embodiments, the memory 644 may also store classification models, identification information for module 630, as well as other information.

In some embodiments, the disclosed methods (e.g., Figures 1-5) may be implemented using software applications that are stored in a memory and executed by a processor (e.g., CPU) provided on the system. In some embodiments, the disclosed methods may be implanted using software applications that are stored in memories and executed by CPUs distributed across the system. As such, the modules of the system may be a general purpose computer system that becomes a specific purpose computer system when executing the routine of the disclosure. The modules of the system may also include an operating system and micro instruction code. The various processes and functions described herein may either be part of the micro instruction code or part of the application program or routine (or combination thereof) that is executed via the operating system. In addition, various other peripheral devices may be connected to the computer platform such as an additional data storage device, a printing device, and other I/O (input/output) devices.

In some embodiments, the analysis device 640 may include a communication interface 646 configured to conduct receiving and transmitting of data between other modules on the system and/or network. The communication interface 646 may be a wired and/or wireless interface, a switched circuit wireless interface, a network of data processing devices, such as LAN, WAN, the internet, or combination thereof. The communication interface may be configured to execute various communication protocols, such as Bluetooth, wireless, and Ethernet, in order to establish and maintain communication with at least another module on the medical facility network.

In some embodiments, the analysis device 640 may include an input/output interface 648 configured for receiving information from one or more input devices 652 (e.g., a keyboard, a mouse, and the like) and/or conveying information to one or more output devices 650 (e.g., a printer, a CD writer, a DVD writer, portable flash memory, etc.).

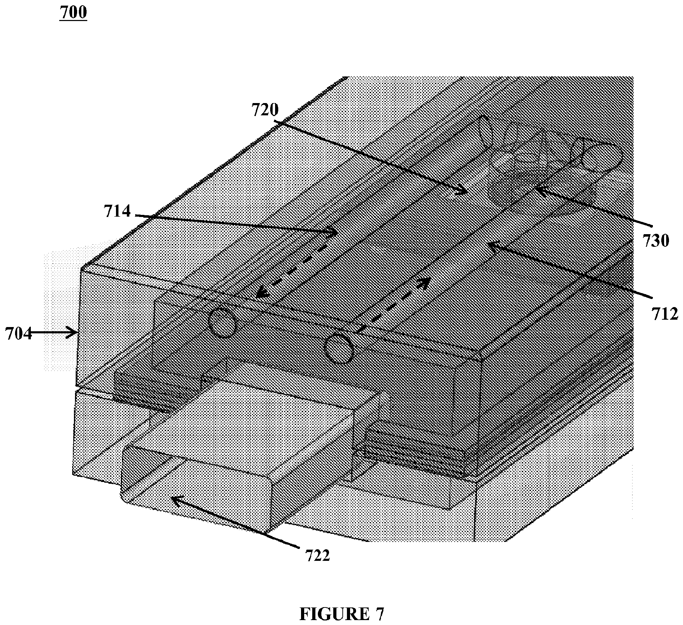

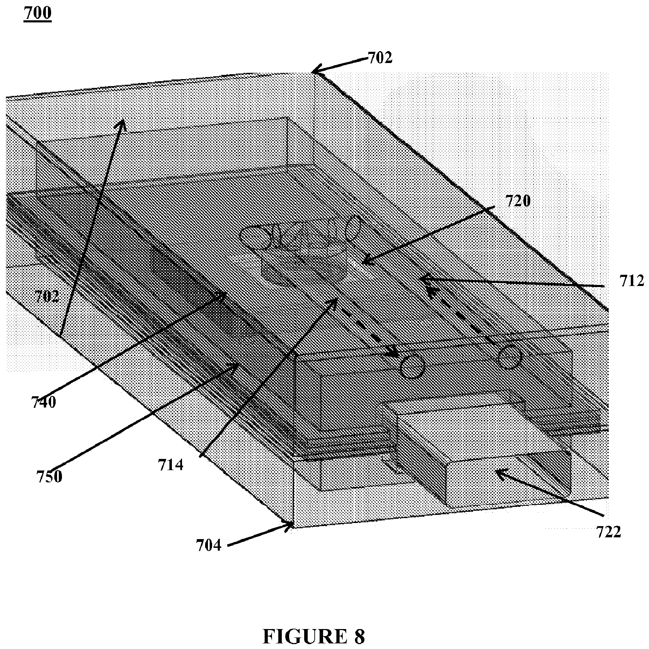

Figures 7-10B show an example of a sensor module and a sensor device according to embodiments. Figures 7-9 show an example of a sensor module 700 according to embodiments.

In some embodiments, the module 700 may include a first end 702 and an opposing second end 704. The module may include at least one fluid connectors disposed at one end. In some embodiments, the module may include an inlet 712 and an outlet 714 disposed at one end. The inlet 712 and outlet 714 may be configured for fluid connection with a sensor device 1000. The inlet 712 and the outlet 714 may include a connector configured to fluidly connect with a sensing device 1000. The inlet 712 and outlet 714 may be configured to extend from one end to a chip 720. In some embodiments, the inlet 712 and 714 may be in fluid communication with the chip 720.

In some embodiments, the sensor module 700 may include a connector 722 configured to connect with a sensing device. In some embodiments, the connector 722 may be a USB connector.

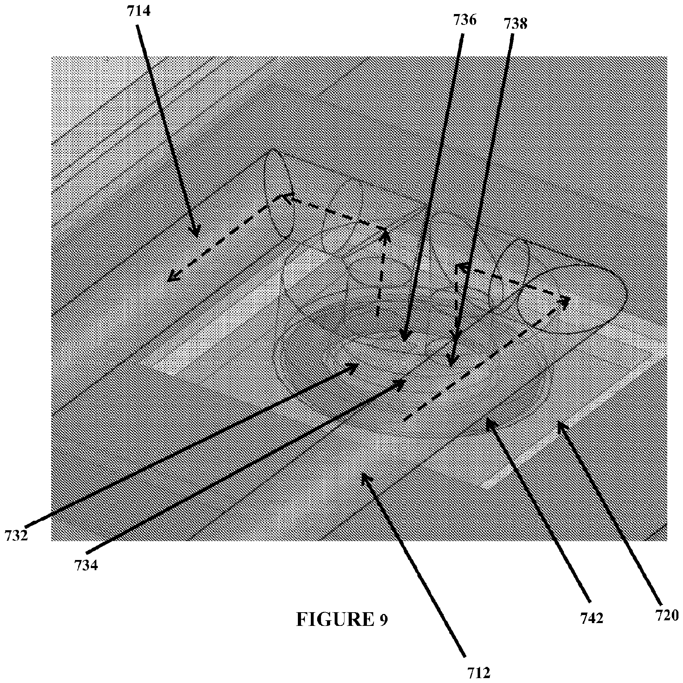

In some embodiments, the chip 720 may include one or more sensors 730. As shown in Figure 9, the chip 720 may include at least four sensors 732, 734, 736, and 738. However, it will be understood that the chip 720 is not limited to four sensors and may include more or less sensors. In some embodiments, the one or more sensors 730 may include one or more antibodies. In some embodiments, the one or more sensors 730 may include at least one housekeeping antibody and at least one sensor target.

In some embodiments, the sensor module 700 may include a component 740 that houses at least the chip 720. In some embodiments, the sensor module 700 may include a circuit board 750. In some embodiments, the circuit board 750 may be a printed circuit board. In some embodiments, the component 740 may be disposed above the circuit board 750.

In some embodiments, the component member 740 may include an isolation member 742 configured to isolate the fluids (e.g., sample, cleaning solution, etc.) flowing through the module and thereby prevent the shorting out the electronics of the circuit board 750. In some embodiments, the isolation member 742 may be an O-ring. In some embodiments, the isolation member 742 may be disposed on the chip and/or configured to surround the sensor 730.

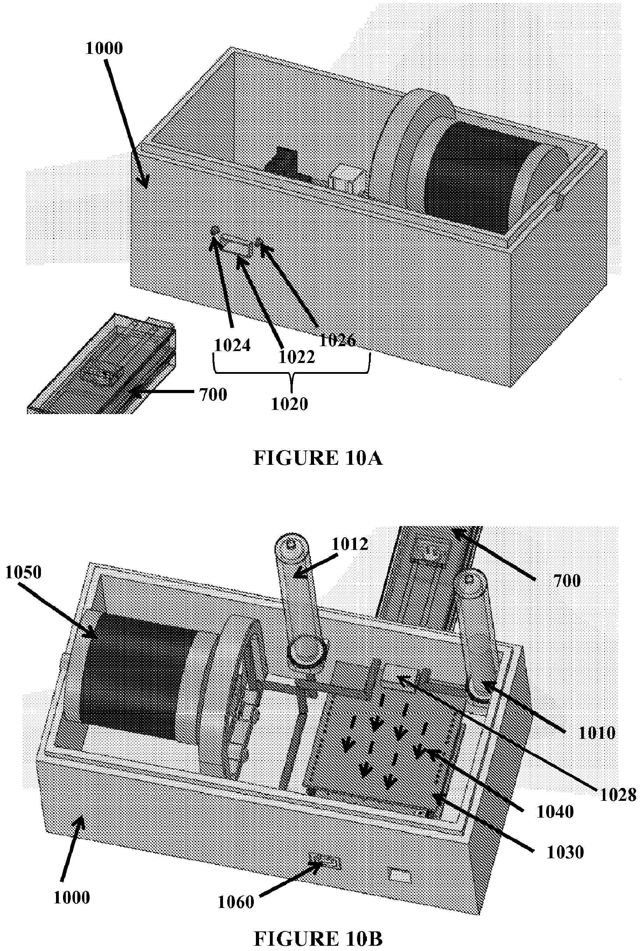

Figures 10A and 10B shows an example of a sensor device 1000. As shown in Figures 10A and 10B, the sensor device 1000 may include a sample chamber or socket 1010 configured to receive a sample. The socket 1010 may also be configured to receive a cleaning sample. In some embodiments, the sensor device 1000 may include a socket 1012 configured to receive the waste from the analysis. In some embodiments, each of the sockets may be configured to receive a sample chamber, such as a tube.

In some embodiments, the sensor device 1000 may also include at least one connector or port 1020 configured to receive a sensor module. In some embodiments, the connectors 1020 may include complimentary connectors (e.g., ports) to the connector 722 and the fluid connectors 712 and 714 of the sensor module 700. The complimentary connectors may include ports. For example, the connectors 1020 may include a port 1022 configured to receive a USB connector and ports 1024 and 1026 configured to respectively receive the fluid connectors 712 and 714 of the sensor module 700. In some embodiments, the sensor device 1000 may include a module interface 1028 configured to conduct receiving and transmitting of data between the sensor module 700 and other modules of the sensor device 1000.

In some embodiments, the sensor device 1000 may include a peristaltic pump 1050. The sensor device 1000 may include a characterization board 1030 configured to extract sensor data (e.g., frequency values) 1040 in substantially real time (e.g., about 10+ data points per second) from the sensor device 700 and transmit the data to the processor.

In some embodiments, the sensor device 1000 may include a connector 1060 configured to connect to another device, for example, an analysis device and/or a display. The connector 1060 may be any port including but not limited to a USB port.

Figure 4 shows an example of a method 400 of operation of the system 600. After a sensor module 630 is loaded into the sensor device 610, the method 400 may include a step 402 of initiate subsystems. The initiation of the operation can be controlled by a computer system, such as the analysis device 640, in response to the end user's commands. After which, the identification information 638 of the sensor module 630 is read (step 404). The microcontroller, such as the processor 614, of the sensor device 610 can respond to the computer's prompting and can provide power to all other subsystems of the device.

Based on the identification information, the appropriate operation parameters may be loaded (step 406). The operation parameters may include the classification model as well as other operation information, such as operation parameters, for obtaining sensor data for the specific biomarker. The operation parameters may be stored in the computer system.

In some embodiments, the operation information can be read from the sensor module 630 through the characterization board 622 to the microcontroller (e.g., the processor 614) and can displayed by both the computer and LED panel (e.g., the display 610). The user may then verify that the module is correct before the operation is initiated (step 408).

After the module is verified (YES at step 408), the operation may be initiated (step 410). The computer system (e.g., the analysis device 640) may send a command to the microcontroller (e.g., the processor 614) of the device 610 to cause the activation of both the characterization board 622 and pump 612 to start the operation (steps 412 and 414). Under computer control, for example, the analysis device 640 can cause the sample can be drawn from a sample chamber and pumped over the sensor module (e.g., sensor module 630). The binding events between the biomarkers in the sample and the antibodies in the sensor module 630 may then be electronically recorded and digitized.

After which, the data may be read and analyzed, for example, by the analysis device 640 (steps 416-422). These steps may be repeated for each data point passed to the computer. The characterization board 622 may read the sensor response (e.g., sensor data including *in-situ* calibration data from a housekeeping protein) (step 416) and may provide the data from the sensor module 630 to the microcontroller, such as the processor 614 (step 418). The data may then be sent to the computer, for example, the analysis device 640, for processing (step 420). The extraction and analysis of the features of the data may then be performed (step 422) by the computer, for example, the analysis device 640. The computer analysis may be done during or after data acquisition. In some embodiments, the analysis may include calibrating the sensor using the calibration data. In some embodiments, the calibrating the sensor may be performed before the analysis of the features of the data.

After the frequency values for each data point are passed to the computer (e.g., the analysis device 640), the pump 612 may be stopped (step 426). The model analysis may then be performed, for example, according to the analysis method 200 (step 428). This may be based on the selected sensor module 630 and operation information. The model analysis may include comparing the acquired data to the respective classification model. The results may then be outputted (step 420). For example, if results may be displayed, printed, stored, and the like. The output may include but is not limited to a certain classification (i.e. patient has likelihood for a disease state), other types of information (i.e. biomarker structure, presence of carcinogen, etc.), as well as a combination thereof. Before the system (e.g., device 610 and/or device 640) is powered down (step 438), the user may be prompted whether additional analysis (e.g., trials) are to be performed (step 432). If additional analysis is to be performed (YES at step 432), then steps 404-438 may be repeated, for example, with another sensor module 630. If the analysis is completed (NO at step 432), then the system may be powered down (step 438).

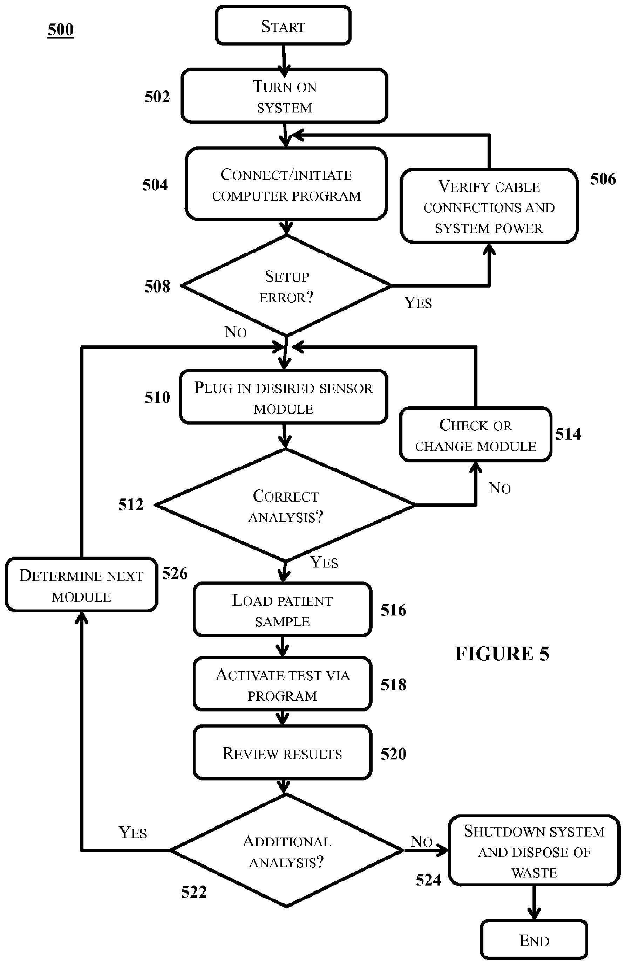

Figure 5 shows a method 500 of classifying a disease state (or condition) and/or determining additional information using a biosensor. As shown in Figure 5, the method 500 may include a step 502 of turning on the device 610. After which, the device 610 may be connected to a computer and/or the analysis program (e.g., analysis device 640) may be initiated (step 504). After, the method 500 may include determining whether the setup is correct (step 508), for example, by the sensor device 610. If an error is determined (YES at step 508), the operator may be alerted and requested to check the set up (step 506). Next, if an error was not determined (NO at step 508), a sensor module 630 may be placed into a sensor device 610 (step 510). For example, if the sensor module 630 includes a USB, the sensor module 630 may be plugged into the sensor device 610.

Next, the method 500 may include a step of determining whether the correct analysis (i.e., sensor module) was selected (step 512). The determining step may include reading the identification information of the sensor module 630 and displaying the corresponding analysis. If the operator determines that the analysis to be performed is incorrect, the operator should check the sensor module 630 to determine if the correct one was selected (NO at step 512). The operator may change the module to a different module 630 (step 514). If it is determined that the sensor module 630 corresponds to the correct analysis (YES at step 512), the patient sample may be loaded (step 516). The test may then be activated in step 518. After the test has been completed, the results may be outputted for review (step 520).

Next, the method 500 may optionally include a step of sterilizing the sensor device. For example, a vial of a cleaning solution may be inserted into the sample chamber and the pump can be caused to draw the solution through the device in order to clean it.

If additional analysis is desired to be performed (YES at step 522), the next module 630 may be determined (step 526) and steps 510-522 may be repeated. If no additional analysis is to be performed (NO at step 522), the system can be shut down and the waste may be disposed (step 524).

It is to be understood that the embodiments of the disclosure may be implemented in various forms of hardware, software, firmware, special purpose processes, or a combination thereof. In one embodiment, the disclosure may be implemented in software as an application program tangible embodied on a computer readable program storage device. The application program may be uploaded to, and executed by, a machine comprising any suitable architecture. The system and method of the present disclosure may be implemented in the form of a software application running on a computer system, for example, a mainframe, personal computer (PC), handheld computer, server, etc. The software application may be stored on a recording media locally accessible by the computer system and accessible via a hard wired or wireless connection to a network, for example, a local area network, or the Internet.

It is to be further understood that, because some of the constituent system components and method steps depicted in the accompanying figures may be implemented in software, the actual connections between the systems components (or the process steps) may differ depending upon the manner in which the disclosure is programmed. Given the teachings of the disclosure provided herein, one of ordinary skill in the related art will be able to contemplate these and similar implementations or configurations of the disclosure.

According to some embodiments, one, some or all components of the sensor system may be structured for single use or be disposable. In some embodiments, one, some or all components may be structured for multi-uses, for example, by being sterilized. According to some embodiments, a portion or combination of the single use items may be sold as a kit.

In some embodiments, the kit may include at least one sensor module according to embodiments. In some embodiments, the kit may include a plurality of sensor modules. The sensor modules may be for the same biomarker(s)/disease state/condition, for different biomarker(s)/disease states/conditions, or a combination thereof. The kit may include at least at least one sensing device according to embodiments. In some embodiments, the kit may include at least one cleaning vial that includes a cleaning solution configured to sterilize the device. In other embodiments, the kit may include a plurality of vials of a cleaning solution.

The disclosure can be better understood with reference to the specific embodiments as set forth in the following example.

## Example 1

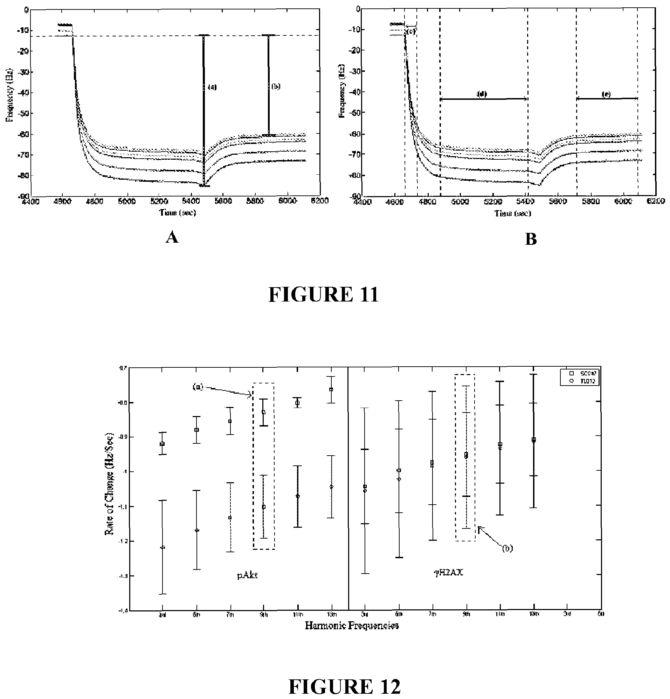

Referring to Figures 11-15, a protocol for creating immunosensor classification models ranging in complexity was developed based on quartz crystal QCM-D frequency shift measurements of an alkanethiol self-assembling monolayer (SAM) functionalized with pAkt, $\gamma$H2AX, $\beta$-Actin, and FITC. Each developed model separated the training set into two classes (HPV positive (SCC47) and HPV negative (TU212) cancer cell lines) based on cancer cell lines. Several pattern recognition algorithms were utilized to develop a class of differentiation models.

### 1.0 Experimental

1.1 Reagents and materials

All antibodies were monoclonal IGg1 for $\beta$-Actin (Sigma-Aldrich), FITC (Santa Cruz), $\gamma$H2AX (Cell Signaling), and pAkt (Cell Signaling). Reactants for the surface chemistry include 3,3'-Dithiodipropionic acid (3,3"-DTP), 1-ethyl-3-(3-dimethylamino-propyl) carbodiimide (EDC) and N-Hydroxysuccinimide (NHS) were obtained from Sigma-Aldrich.

Squamous cell carcinoma of the head and neck (SCCHN) human cell line TU212 used for this study was established from a primary hypopharyngeal tumor. It was obtained from Dr. Gary L. Clayman's laboratory (The University of Texas M.D. Anderson Cancer Center, Houston, TX). The HPV-16-positive SCCHN cell line UM-SCC47 was obtained from Dr. Thomas Carey, University of Michigan. The tumor cell line was grown in DMEM/F12 (1:1) with supplemented 5% fetal bovine serum.

1.2 Antibody immobilization protocol

All Au-sensors were exposed to UV/Ozone treatment for 10 minutes, then submerged in a $H_2O/H_2O_2/NH_4$ (5:1:1) heated 75°C for 5 minutes, and subsequently rinsed with DI water and exposed to UV/Ozone treatment for an additional 10 minutes.

Once housed in the QCM-D chambers, each sensor's resonant frequency was obtained using DI water as the initial surface medium. A self-assembled monolayer was then constructed on the surface of each sensor using 0.01M 3,3'-DTP in ethanol, and of (_)M EDC/NHS (Prime & Whitesides, 1991). See Prime, K. L., & Whitesides, G. M. (1991). Self-assembled organic monolayers: model systems for studying adsorption of proteins at surfaces. Science (New York, N.Y.), 252(5010), 1164-1167. Each of the four sensors was then inoculated with a different antibody (_-Actin, FITC, _H2AX, and pAkt) at a concentration of 10 ng/ml.

1.3 Cell line preparation

Whole cell lysatcs were extracted using lysis buffer containing 50mM Tris-Cl (pH 8.0), 150mM NaCl, 0.02% Sodium azide, 0.2% SDS, 2% Igepal CA-630, 0.5% sodium deoxycholate and protease inhibitor cocktail (Sigma Aldrich). Total protein content in the lysates was determined using the Bio-Rad protein assay (Bio-Rad).

1.4 QCM measurements

All trials were conducted in the flow chambers of the Q-Sense E4 module (Biolin Scientific/QSense). The module records the frequency shift and dissipation of each chamber and displays the data in real-time. After antibody immobilization and a PBS buffer wash off, the same lysate sample (1:20 dilution in PBS) was flown across all the sensors followed by a final PBS buffer wash off.

**2.0 Results and discussion**

2.1 Feature selection

Raw immunosensor data has no beneficial information for analysis without the definition of discrete features. Feature extraction is the process of obtaining qualitative values from raw data. A typical feature for immunosensors is denoted by tracking the change in the detection mechanism. For piezoelectric biosensors, this would be a shift in the device's fundamental harmonic frequencies. The primary feature is the frequency shift of the total bonded antigen, based on the initial frequency after antibody immobilization to the final PBS wash off which is seen in Figure 11(b). The maximum to minimum frequency shift over the period of lysate exposure is shown in Figure 11(a).

Previous studies have utilized the slope of resonant frequency shifts as a data feature in association with the antibody affinity and dissociation constants for the target antigen (Skládal, 2009). See Skladal, P. (2009). Piezoelectric Quartz Crystal Resonators Applied for Immunosensing and Affinity Interaction Studies. Biosensors and Biodetection Methods and Protocols, 2, 37-50. Poitras & Tufenkji (2009) developed a rapid detection biosensor for Escherichia coli (E. coli) utilizing the frequency shift within the first few minutes of sample exposure, focusing solely on the initial loading event. See Poitras, C., & Tufenkji, N. (2009). A QCM-D-based biosensor for E. coli O157:H7 highlighting the relevance of the dissipation slope as a transduction signal. Biosensors and Bioelectronics, 24(7), 2137-2142. Three regions of interest have been defined in which linear regression models were fitted to determine the slope. As shown in Figure 11B, each region correlates to the binding events between the antibody and target antigen [binding affinity (11(c)), equilibrium (11(d)), and dissociation (11(e)].

2.2 Two dimensional Linear Classifier

After the selection of the features, three trials for each lysate sample (6 trials total) were evaluated as the "training" data set for developing the classification models. Each trial's feature set is then normalized based on the value of the negative control (FITC) in order to remove the effects of non-specific binding (Eq. 1). Two features were selected based on minimal standard deviations and maximum separation from the mean values ((a) in Figure 12). Fig. 12 shows an example mean and standard deviation values for binding affinity region slope values, (a) Shows a desired data set separation, optimal for developing a model. (b) An undesired case where there is no statistical separation between the sample types.

$$X_{Final} = X_{ij} - X_{kj}$$
$$i = \{\beta\text{-Actin}, \gamma\text{H2AX}, pAkt\}, j = \{3^{rd}, 5^{th}, ..., 13^{th}\}, k = FITC$$

$$\ldots\ldots\ldots\ldots(1)$$

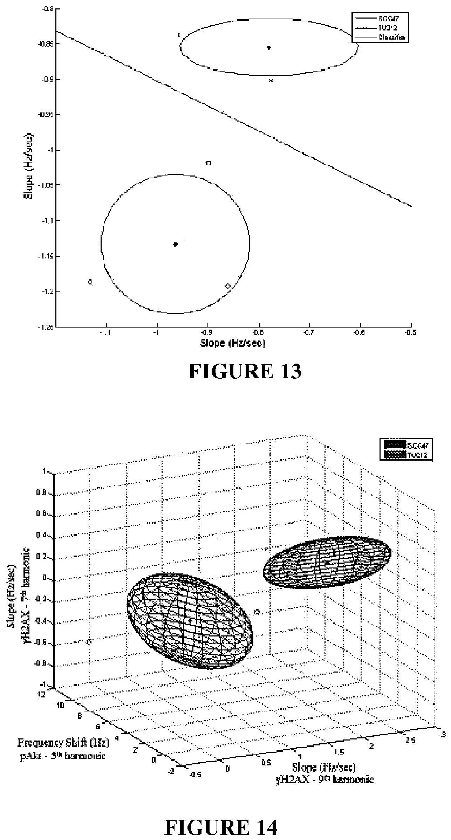

Before normalization, the binding affinity region slope values showed significant separation. Therefore, the 11th harmonic values for $\beta$-Actin and 7th harmonic values for pAkt were selected to develop the model. Gaussian regions were plotted based on the selected features mean and standard deviations values (Fig. 13). These regions define areas in which there is a high probability that subsequent trial frequency values would fall. The Kozinec's algorithm was then applied to define a linear classifier model (equation 2).

$$q(x) = \begin{cases} 1 & \text{if } f(x) = (w \cdot x) + b \geq 0, \\ 2 & \text{if } f(x) = (w \cdot x) + b < 0, \end{cases} \qquad \ldots\ldots\ldots\ldots(2)$$

The unknown variables defined by the classifier algorithm are the parameter vector $\omega$ (dim x 1) and the bias b (1 x 1), where dim is the level of dimensionality of the model (i.e., 2D) (Franc & Hlavác, 2004).

Fig. 13 shows 2D Model plot based on the binding affinity region slope values. X-axis is the 11th harmonic values for β-Actin and the y-axis is the 7th harmonic values for pAkt. The parameter vector and bias values are $\omega$= [0.051, 0.143] and b = 0.180, respectively.

2.3 Three dimensional Linear Classifier

As mentioned above in Section 2.2, following negative control normalization there was no noticeable separation for the remaining features. Yet, it can be noted that there are similar trends for several of the antigen expressions across harmonic values. This alludes to more analytical details that have yet to be utilized. In the field of molecular biology, measuring gene expression entails determining the amount of a gene product (usually proteins) from a crude cell extract. This is then normalized by a positive control which is a gene product that is equally expressed in all samples. In this case, β-Actin serves as the positive control and the rest of the data is normalized based on the mean value of all the harmonic values for each individual trial (Eq. 3).

$$\mu = \Sigma k_j, \ X_{final} = X_{ij} / \mu$$
$$i = (\gamma H2AX, pAkt), j = (3^{rd}, 5^{th}, \ldots, 13^{th}), k = \beta\text{-Actin} \qquad \ldots\ldots\ldots\ldots\ldots\ldots(3)$$

Based on this normalization, the data now shows smaller standard deviations and greater separation between mean values. If the standard deviations are overlapped for more than one of the selected features then increasing the dimensionality of the model is necessary. After both positive and negative normalizations - γH2AX (5th harmonic, equilibrium slope), pAkt (5th harmonic, total _f), and γH2AX (7th harmonic, dissociation slope) were selected for developing the model (Fig. 14) - the Kozinec's algorithm was incorporated to define a linear classifier model

Fig. 14 shows a 3D model plot. X-axis are X-axis are equilibrium region slope γH2AX 9th harmonic values, y-axis are total frequency shift pAkt 5th harmonic values, and z-axis are dissociation region slope γH2AX 7th harmonic values. The parameter vector and bias values are $\omega$ =[0.243, -0.125, 0.551] and b = -0.425, respectively.

2.4. Bayesian Classifier

In order to develop a more complex classification model a Bayesian decision rule algorithm with Gaussian mixer models were incorporated. Although this model is capable of separating highly correlated feature sets, for simplicity and visualization purposes, a linearly separable training set with a training set of 30 values was selected.

The training set for this model was extracted from the harmonic frequency values of the binding affinity slope in addition to the respective harmonic frequency values of the equilibrium slope for γH2AX. Only the 3rd through the 11th harmonic values were included due to the poor regression fits for the 13th harmonic equilibrium region slope values (0.75± 0.11). This feature set was selected to show the model's ability for class differentiation with only utilizing the normalize feature values of one target molecule.

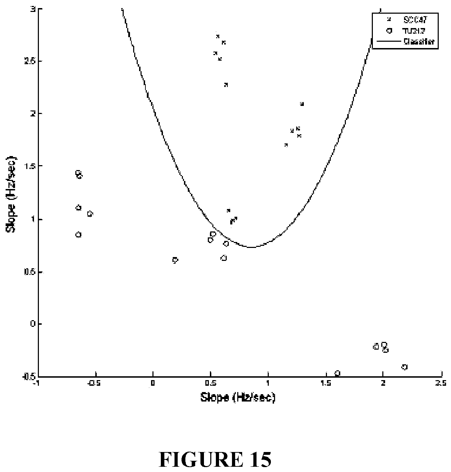

Figure 15 shows a Bayesian classifier based on Guassian Mixture Models (GMM). X-axis are binding affinity region slope values and the y-axis are the equilibrium region slope values for the 3rd through 11th harmonics.

For this study, the differentiation for the binary case (two classes) with lower dimension feature sets (i.e. 2D, 3D) was focused on, although each of these models are capable of differentiating multi-class scenarios with higher feature set dimensionality.

While the disclosure has been described in detail with reference to exemplary embodiments, those skilled in the art will appreciate that various modifications and substitutions may be made thereto without departing from the spirit and scope of the disclosure as series forth in the appended claims. For example, elements and/or features of different exemplary embodiments may be combined with each other and/or substituted for each other within the scope of this disclosure and appended claims.

The invention is further defined in the following numbered embodiments:

1. A method of quantifying a sample using a sensor, the method comprising:

   obtaining sensor data, the sensor data including *in-situ* calibration data from a housekeeping protein;
   analyzing the sensor data; and
   outputting results of the analyzing.

2. The method of clause 1, wherein the analyzing the sensor data includes:

   determining quantitative values from the sensor data; and
   comparing the quantitative values to a classification model.

3. The method of clause 1, wherein the determining quantitative values includes determining frequency values.

4. The method of clause 1, wherein the sample is a blood sample from a subject and the housekeeping protein is β-Actin.

5. A module configured to be used with a sensing device that is configured to analyze a sample, the module comprising:

   a chip including at least one sensor target to be tested;
   at least one fluid connector configured for fluid communication with a sensing device; and
   a connector configured to connect the module to a sensing device.

6. The module of clause 5, wherein the connector includes a USB connector.

7. The module of clause 5, further comprising:
identification information, the identification information including operation information.

8. The module of clause 5, wherein the identification information includes a bar code.

9. The module of clause 5, wherein the chip includes one or more antibodies.

10. The module of clause 5, wherein the chip includes at least one sensor.

11. A system configured to analyze a sample, comprising:

   a sensing device configured to extract data from at least one module, the device including:

      at least one port configured to receive a module; and
      a controller configured to control an operation of the sensing device; and

   at least one module, the module including:

      a chip including at least one sensor target to be tested;
      at least one fluid connector configured for fluid communication with the sensing device; and
      a connector configured to connect the module to the sensing device.

12. The system of clause 11, further comprising:
an analysis device configured to perform analysis of the data.

13. The system of clause 12, wherein the analysis includes quantification of frequency values.

14. The system of clause 11, wherein the module includes identification information, the identification information includes operation information.

15. The system of clause 14, wherein the sensing device is configured to operate according to the operation information.

16. The system of clause 11, wherein the sensing device includes a characterization board configured to extract the data in substantially real time.

17. The system of clause 16, wherein the data includes frequency values and in-situ calibration data.

18. The system of clause 11, wherein:

the connector of the module includes a USB connector; and
the at least one port includes a port configured to receive the connector and at least one port configured to receive the at least one fluid connector of the module.

19. The system of clause 11, wherein the chip includes one or more antibodies.

20. The system of clause 11, wherein the chip includes at least one sensor.

**Claims**

1. A module configured to be used with a sensing device that is configured to analyse a sample, the module comprising:

a chip including at least one piezoelectric sensor comprising at least one antibody for at least one biomarker in the sample
a circuit board; and
a connector configured to connect the module to the sensing device.

2. The module of claim 1, further comprising identification information, the identification information including operation information.

3. The module of claim 1 or 2, wherein the at least one biomarker includes one or more antibodies.

4. A system configured to analyse a sample, comprising:

a sensing device configured to extract data from at least one module, the device including: at least one port configured to receive the at least one module; and
a controller configured to control an operation of the sensing device; and the at least one module including:

a chip including one or more sensors, wherein at least one of the sensors is a piezoelectric sensor comprising at least one antibody for at least one biomarker in the sample; and
a connector configured to connect the module to the sensing device.

5. The system of claim 4, further comprising:
an analysis device configured to perform analysis of the data to determine a presence or concentration of the at least one biomarker in the sample.

6. The system of claim 5, wherein the analysis device includes one or more processors.

7. The system of claim 5 or 6, wherein the analysis includes quantification of frequency values wherein the frequency values for the at least one piezoelectric sensor include a shift in at least one of the fundamental and harmonic frequencies of the device.

8. The system of any of claims 4-7, wherein:

the module includes identification information, wherein the identification information includes at least one of: the type of analysis to be performed, operation parameters, and the classification model related to the analysis; and
the sensing device is configured to operate according to the operation parameters.

9. The system of any one of claims 4-8, wherein the sensing device includes a characterization board configured to extract the data in substantially real time.

10. The system of any one of claims 4-9, wherein the data includes:

> frequency values, wherein the frequency values for the at least one piezoelectric sensor include a shift in at least one of the fundamental and harmonic frequencies of the device; and
> in-situ calibration data.

11. Use of the module according to any of claims 1-3, or the system according to any of claims 4-10, in a method of determining the presence or concentration of at least one biomarker in a sample obtained from a subject.

12. The use of a module or system according to claim 11, wherein determining the presence or concentration of at least one biomarker provides a diagnosis of a disease in a subject or provides for evaluating effectiveness of a treatment for a disease.

13. The use of the module or system according to claim 11 or 12, wherein the sensor can be calibrated by measuring a housekeeping protein.

14. The use of the module or system according to claim 12, wherein the sample is a blood sample from a subject and the housekeeping protein is $\beta$-Actin.

100

```
        ┌─────────────────┐
        │      START       │
        └─────────────────┘
                 │
                 ▼
        ┌─────────────────┐
  102   │    CALIBRATE     │
        │      SENSOR      │
        └─────────────────┘
                 │
                 ▼
        ┌─────────────────┐
  104   │  OBTAIN SENSOR   │
        │       DATA       │
        └─────────────────┘
                 │
                 ▼
        ┌─────────────────┐
  106   │     ANALYZE      │
        │     FEATURES     │
        └─────────────────┘
                 │
                 ▼
        ┌─────────────────┐
  108   │  OUTPUT RESULTS  │
        └─────────────────┘
                 │
                 ▼
        ┌─────────────────┐
        │       END        │
        └─────────────────┘
```

**FIGURE 1**

**200**

```
┌─────────────────────┐
│                     │
│       START         │
│                     │
└─────────────────────┘
          │
          ▼
┌─────────────────────┐
│     DETERMINE       │
│   QUANTITATIVE      │
│  VALUES FROM DATA   │
└─────────────────────┘
          │
          ▼
┌─────────────────────┐
│ COMPARE DATA TO A   │
│  CLASSIFICATION     │
│      MODEL          │
└─────────────────────┘
          │
          ▼
┌─────────────────────┐
│                     │
│        END          │
│                     │
└─────────────────────┘
```

202

204

**FIGURE 2**

**300**

```
                          ┌─────────┐
                          │  START  │
                          └─────────┘
                               │
           302    ┌──────────────────────────┐
                  │        DETERMINE         │
                  │         DESIRED          │
                  │     DISEASE/CONDITION    │
                  └──────────────────────────┘
                                              306
     ┌────────────────────┐         ┌────────────────────┐
     │     ESTABLISH      │         │     DETERMINE      │
304  │  TRAINING/TESTING  │         │     POTENTIAL      │
     │ PATIENT POPULATION │         │    BIOMARKERS      │
     └────────────────────┘         └────────────────────┘
                      ┌──────────────────────┐
                      │    RUN TRIALS FOR     │
                      │        EACH           │
                      │  PATIENT/BIOMARKER    │  308
                      └──────────────────────┘
                      ┌──────────────────────┐
                      │ INSPECT DATA VALUES   │
                      │   FOR STATISTICAL     │  310
                      │     SEPARATION        │
                      └──────────────────────┘
                      ┌──────────────────────┐
                      │   SELECT PATTERN      │
                      │   RECOGNITION         │
                      │     MODELS            │  312
                      │ FOR CLASSIFICATION    │
                      └──────────────────────┘

      < 95%      ╱  TEST           ╲   314    < 85%
               ╱  CLASSIFICATION    ╲
                ╲    MODELS         ╱
                 ╲                 ╱
                      │  > 95%          ┌──────────────────┐
                      │                 │     OUTPUT       │
                      │                 │  CLASSIFICATION  │
                ┌─────────┐             │     MODELS       │
                │   END   │  316        └──────────────────┘
                └─────────┘
```

**FIGURE 3**

**400**

```
                        ┌──────────────┐
                        │    START     │
                        └──────┬───────┘
                               ▼
                        ┌──────────────┐
                        │   INITIATE   │  402
                        │  SUBSYSTEMS  │
  ┌──────────┐          └──────┬───────┘
436│ RESTART  │────────────────┤
  └──────────┘                 ▼
                        ┌──────────────────┐
                        │ READ SENSOR MODULE│ 404
                        │      TYPE         │
                        └────────┬──────────┘
                                 ▼
                        ┌──────────────────┐
                        │ LOAD OPERATION    │ 406
                        │ PARAMETERS/       │
                        │ INFORMATION       │
                        └────────┬──────────┘
```

CORRECT ANALYSIS? 408 — NO → END

YES

INITIATE OPERATION 410

412 ACTIVATE CHARACTERIZATION BOARD    START THE PUMP 414

FIGURE 4

416 READ SENSOR RESPONSE

418 PASS FREQUENCY VALUES TO MICROCONTROLLER

420 PASS FREQUENCY VALUES TO COMPUTER

422 FEATURE EXTRACTION AND ANALYSIS

END

436 RESTART

426 STOP PUMP

YES

ADDITIONAL ANALYSIS? — NO → POWER DOWN SYSTEMS 438

428 MODEL ANALYSIS → 420 OUPUT RESULTS → 432

**500**

```
                              ┌─────────────┐
                              │    START    │
                              └─────────────┘
                                     │
                                     ▼
        502              ┌─────────────────────┐
                         │      TURN ON        │
                         │      SYSTEM         │
                         └─────────────────────┘
                                     │
                                     ▼
        504         ┌─────────────────────┐        ┌──────────────────────┐
                    │  CONNECT/INITIATE    │        │   VERIFY CABLE       │  506
                    │  COMPUTER PROGRAM    │        │  CONNECTIONS AND     │
                    └─────────────────────┘         │   SYSTEM POWER       │
                                     │              └──────────────────────┘
                                     ▼                        ▲
        508            ◇ SETUP ERROR? ◇──── YES ──────────────┘
                                     │
                                    NO
                                     │
                                     ▼
        510         ┌─────────────────────┐
                    │ PLUG IN DESIRED      │        ┌──────────────────────┐
                    │   SENSOR MODULE      │        │   CHECK OR           │  514
                    └─────────────────────┘         │  CHANGE MODULE       │
                                     │              └──────────────────────┘
                                     ▼                        ▲
        512            ◇ CORRECT ANALYSIS? ◇──── NO ──────────┘
                                     │
                                    YES
  ┌──────────────────┐              │
  │ DETERMINE NEXT   │ 526          ▼
  │    MODULE        │    ┌─────────────────────┐
  └──────────────────┘    │    LOAD PATIENT     │  516
                          │      SAMPLE         │
                          └─────────────────────┘
                                     │
                                     ▼
                          ┌─────────────────────┐
                          │  ACTIVATE TEST VIA  │  518
                          │      PROGRAM        │
                          └─────────────────────┘
                                     │
                                     ▼
                          ┌─────────────────────┐
                          │   REVIEW RESULTS    │  520
                          └─────────────────────┘
                                     │
                                     ▼
            YES ──── ◇ ADDITIONAL ANALYSIS? ◇ ──── NO ── ┌──────────────────┐
                                                          │ SHUTDOWN SYSTEM  │
                       522                                │ AND DISPOSE OF   │ 524
                                                          │     WASTE        │
                                                          └──────────────────┘
                                                                   │
                                                                   ▼
                                                          ┌─────────────┐
                                                          │     END     │
                                                          └─────────────┘
```

**FIGURE 5**

600

| | |
|---|---|
| 612 | PUMP |
| | 616 MEMORY |
| | DISPLAY 610 |
| 614 | PROCESSOR |
| | 618 CONNECTOR(S) 624 |
| 620 | MODULE INTERFACE |
| | CHARACTERIZATION BOARD 622 |

SENSOR DEVICE

| | |
|---|---|
| 632 | FLUID CONNECTORS 630 |
| 634 | CHIP |
| 636 | CONNECTOR |
| 638 | ID |

SENSOR MODULE

| | |
|---|---|
| 642 | PROCESSOR 640 |
| 644 | MEMORY |
| 646 | COMMUNICATION INTERFACE |
| 648 | I//O INTERFACE |

ANALYSIS DEVICE

| | |
|---|---|
| 650 | OUTPUT DEVICE(S) |
| 652 | INPUT DEVICE(S) |

FIGURE 6

<u>700</u>

720

714

730

712

704

722

**FIGURE 7**

700

**FIGURE 8**

**FIGURE 9**

1000

1024  1022  1026

700

1020

**FIGURE 10A**

1050

1012

700

1010

1028

1040

1030

1000  1060

**FIGURE 10B**

**FIGURE 11**

**FIGURE 12**

**FIGURE 13**

**FIGURE 14**

**FIGURE 15**

Europäisches
Patentamt

European
Patent Office

Office européen
des brevets

# EUROPEAN SEARCH REPORT

Application Number

EP 21 16 6730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | J. WIEST ET AL: "Intelligent Mobile Lab for Metabolics in Environmental Monitoring", ANALYTICAL LETTERS, vol. 39, no. 8, 1 July 2006 (2006-07-01), pages 1759-1771, XP055290381, ISSN: 0003-2719, DOI: 10.1080/00032710600714089 * pg 1761-1767, fig 1, 3, 4. * | 1-14 | INV. G01N33/53 G01N33/48 G01N33/68 |
| A | Eléonore Cabala: "Monitoring multiparametrischer komplexer Mikrosensorarrays für zelluläre Analytik", , 29 January 2007 (2007-01-29), XP055290422, Retrieved from the Internet: URL:https://mediatum.ub.tum.de/doc/620614/620614.pdf [retrieved on 2016-07-21] * pg 30-33; fig 3.9. * | 1-14 | |
| A | US 7 029 855 B1 (TRAN NATHANIEL TUE [US]) 18 April 2006 (2006-04-18) * pg 30-33; fig 3.9. * | 1-14 | TECHNICAL FIELDS SEARCHED (IPC) G01N G06F |
| A | WO 2009/076551 A2 (UNIV CALIFORNIA [US]; DAS DEBOPRIYA [US]; GRAY JOE [US]; WANG NICHOLAS) 18 June 2009 (2009-06-18) * par 0087. * | 1-14 | |
| Y | US 2012/209098 A1 (GOODE PAUL V JR [US] ET AL) 16 August 2012 (2012-08-16) * par 0028, 0311, 0308, 0305, 0035-0037. * | 1-14 | |
| A | US 2008/020409 A1 (PAWLAK MICHAEL [DE] ET AL) 24 January 2008 (2008-01-24) * the whole document * | 1-14 | |

-/--

The present search report has been drawn up for all claims

| Place of search | Date of completion of the search | Examiner |
|---|---|---|
| Munich | 31 August 2021 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 16 6730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2010/145317 A1 (LASTER MORRIS [IL] ET AL) 10 June 2010 (2010-06-10) * par 0098, 0126. * ----- | 1-14 | |

TECHNICAL FIELDS
SEARCHED      (IPC)

The present search report has been drawn up for all claims

| Place of search | Date of completion of the search | Examiner |
|---|---|---|
| Munich | 31 August 2021 | Motrescu-Hateley, E |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 21 16 6730

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2021

| Patent document cited in search report | Publication date | Patent family member(s) | Publication date |
|---|---|---|---|
| US 7029855 B1 | 18-04-2006 | NONE | |
| WO 2009076551 A2 | 18-06-2009 | US 2009177450 A1 | 09-07-2009 |
| | | WO 2009076551 A2 | 18-06-2009 |
| US 2012209098 A1 | 16-08-2012 | EP 1648293 A1 | 26-04-2006 |
| | | EP 2494921 A2 | 05-09-2012 |
| | | EP 2494922 A2 | 05-09-2012 |
| | | EP 2497415 A1 | 12-09-2012 |
| | | EP 2497420 A1 | 12-09-2012 |
| | | EP 2497421 A1 | 12-09-2012 |
| | | EP 2508129 A1 | 10-10-2012 |
| | | JP 5037128 B2 | 26-09-2012 |
| | | JP 6803945 B2 | 23-12-2020 |
| | | JP 2007501028 A | 25-01-2007 |
| | | JP 2008096448 A | 24-04-2008 |
| | | JP 2012016597 A | 26-01-2012 |
| | | JP 2015061662 A | 02-04-2015 |
| | | JP 2017074483 A | 20-04-2017 |
| | | JP 2019181215 A | 24-10-2019 |
| | | US 2005027180 A1 | 03-02-2005 |
| | | US 2005027181 A1 | 03-02-2005 |
| | | US 2005027462 A1 | 03-02-2005 |
| | | US 2005027463 A1 | 03-02-2005 |
| | | US 2005187720 A1 | 25-08-2005 |
| | | US 2006040402 A1 | 23-02-2006 |
| | | US 2008021666 A1 | 24-01-2008 |
| | | US 2008183061 A1 | 31-07-2008 |
| | | US 2008183399 A1 | 31-07-2008 |
| | | US 2008189051 A1 | 07-08-2008 |
| | | US 2008194936 A1 | 14-08-2008 |
| | | US 2008194937 A1 | 14-08-2008 |
| | | US 2008195967 A1 | 14-08-2008 |
| | | US 2008306368 A1 | 11-12-2008 |
| | | US 2009012379 A1 | 08-01-2009 |
| | | US 2010161269 A1 | 24-06-2010 |
| | | US 2010174167 A1 | 08-07-2010 |
| | | US 2010217555 A1 | 26-08-2010 |
| | | US 2010217557 A1 | 26-08-2010 |
| | | US 2011231141 A1 | 22-09-2011 |
| | | US 2012209098 A1 | 16-08-2012 |
| | | US 2012215461 A1 | 23-08-2012 |
| | | WO 2005011489 A1 | 10-02-2005 |
| US 2008020409 A1 | 24-01-2008 | AU 2004318038 A1 | 13-10-2005 |
| | | CA 2559803 A1 | 13-10-2005 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 21 16 6730

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2021

| Patent document cited in search report | Publication date | Patent family member(s) | | Publication date |
|---|---|---|---|---|
| | | CN | 1954213 A | 25-04-2007 |
| | | EP | 1730530 A1 | 13-12-2006 |
| | | JP | 2007526460 A | 13-09-2007 |
| | | US | 2008020409 A1 | 24-01-2008 |
| | | WO | 2005095965 A1 | 13-10-2005 |
| US 2010145317 A1 | 10-06-2010 | AU | 2008242142 A1 | 30-10-2008 |
| | | CA | 2684457 A1 | 30-10-2008 |
| | | CN | 101715317 A | 26-05-2010 |
| | | EP | 2139393 A2 | 06-01-2010 |
| | | JP | 2010524554 A | 22-07-2010 |
| | | US | 2010145317 A1 | 10-06-2010 |
| | | WO | 2008129532 A2 | 30-10-2008 |

EPO FORM P0459

For more details about this annex : see Official Journal of the European Patent Office, No. 12/82

page 2 of 2

## REFERENCES CITED IN THE DESCRIPTION

**Patent documents cited in the description**

- WO 61751107 A

- WO 61784151 A

**Non-patent literature cited in the description**

- **HOLFORD, T. R. ; DAVIS, F. ; HIGSON, S. P.** Recent trends in antibody based sensors. *Biosensors and Bioelectronics,* 2012, vol. 34 (1), 12-24
- **PRIME, K. L. ; WHITESIDES, G. M.** Self-assembled organic monolayers: model systems for studying adsorption of proteins at surfaces. *Science,* 1991, vol. 252 (5010), 1164-1167

- **SKLADAL, P.** Piezoelectric Quartz Crystal Resonators Applied for Immunosensing and Affinity Interaction Studies. *Biosensors and Biodetection Methods and Protocols,* 2009, vol. 2, 37-50
- **POITRAS, C. ; TUFENKJI, N.** A QCM-D-based biosensor for E. coli O157:H7 highlighting the relevance of the dissipation slope as a transduction signal. *Biosensors and Bioelectronics,* 2009, vol. 24 (7), 2137-2142